(19) Europäisches Patentamt
European Patent Office
Office européen des brevets

(11) EP 3 522 880 B1

(12) EUROPEAN PATENT SPECIFICATION

(45) Date of publication and mention of the grant of the patent:
**02.12.2020 Bulletin 2020/49**

(21) Application number: **17787773.5**

(22) Date of filing: **05.10.2017**

(51) Int Cl.:
*A61K 31/4439* (2006.01) *A61K 31/4192* (2006.01)
*A61K 31/192* (2006.01) *A61P 13/12* (2006.01)

(86) International application number:
**PCT/US2017/055400**

(87) International publication number:
**WO 2018/067857 (12.04.2018 Gazette 2018/15)**

# (54) METHODS OF TREATING ACUTE KIDNEY INJURY

VERFAHREN ZUR BEHANDLUNG EINER AKUTEN NIERENVERLETZUNG

MÉTHODE DE TRAITEMENT DE LÉSIONS RÉNALES AIGUËS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.10.2016 US 201662404390 P**

(43) Date of publication of application:
**14.08.2019 Bulletin 2019/33**

(73) Proprietor: **Mitobridge, Inc.**
**Cambridge, MA 02138 (US)**

(72) Inventors:
- **LAGU, Bharat**
  **Acton, MA 01720 (US)**
- **PATANE, Michael**
  **Andover, MA 01810 (US)**
- **TOZZO, Effie**
  **Lexington, MA 02420 (US)**
- **TRZASKA, Scott**
  **Raritan, NJ 08869 (US)**

(74) Representative: **Potter Clarkson**
**The Belgrave Centre**
**Talbot Street**
**Nottingham NG1 5GG (GB)**

(56) References cited:
**WO-A1-2017/062468**

- **DEDOROVA ET AL.: "PEROXISOME PROLIFERATOR-ACTIVATED RECEPTOR DELTA AGONIST, HPP593, PREVENTS RENAL NECROSIS UNDER CHRONIC ISCHEMIA", PLOS ONE, vol. 8, no. 5, E64436, May 2013 (2013-05), XP002776109,**
- **EUN YOUNG LEE ET AL.: "PEROXISOME PROLIFERATOR-ACTIVATED RECEPTOR-DELTA ACTIVATIO AMELIORATES ALBUMINURIA BY PREVENTING NEPHRIN LOSS AND RESTORING PODOCYTE INTEGRITY IN TYPE 2 DIABETES", NEPHROL. DIAL. TRANSPLANT., vol. 27, 2012, pages 4069-4079, XP002776110,**
- **LETAVERNIER ET AL.: "PEROXISOME PROLIFERATOR-ACTIVATED RECEPTOR BETA/DELTA EXERTS A STRONG PROTECTION FROM ISCHEMIC ACUTE RENAL FAILURE", J. AM. SOC. NEPHROL., vol. 16, 2005, pages 2395-2402, XP002776111,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 522 880 B1

## Description

### FIELD OF THE INVENTION

The present invention is directed to methods of treating a human patient with acute kidney injury.

### BACKGROUND OF THE INVENTION

Acute kidney injury (AKI), previously known as acute renal failure (ARF), is a clinical syndrome characterized by rapid deterioration of renal function that occurs within days. The principal feature of AKI is an abrupt decline in glomerular filtration rate (GFR), resulting in the retention of nitrogenous wastes (urea, creatinine). In the general world population, 170-200 cases of severe AKI per million population occur annually. To date, there is no specific treatment for established AKI. Several drugs have been found to prophylactically ameliorate toxic and ischemic experimental AKI, as manifested by lower serum creatinine levels, reduced histological damage and faster recovery of renal function in different animal models. These include anti-oxidants, calcium channel blockers, diuretics, vasoactive substances, growth factors, anti-inflammatory agents and more. However, these drugs have been studied in clinical trials and showed no benefit, and their use in clinical AKI has not been approved. Treatment once AKI has developed is even more difficult.

Fedorova et al. (PLOS ONE 8 (2013), e64436) describes that an agonist of PPAR delta, HPP 593, prevents renal necrosis under chronic ischemia, which corresponds to what happens during acute kidney injury.

Eun Young Lee et al. (Nephrol. Dial. Transplant. 27 (2012), 4069-4079) describes the efficacy of GW617042, an agonist for PPAR-δ for the treatment of diabetic nephropathy.

The compounds disclosed herein are exemplary modulators of peroxisome proliferator-activated receptor delta (PPARδ). PPARδ is a nuclear receptor that is capable of regulating mitochondria biosynthesis. Modulating the activity of PPARδ has been shown to be useful for the treatment of diseases.

### SUMMARY OF THE INVENTION

It has now been found that a compound, (*R*)-3-methyl-6-(2-((5-methyl-2-(6-(trifluoromethyl)pyridin-3-yl)-1*H*-imidazol-1-yl)methyl)phenoxy)hexanoic acid, represented by the following structural formula:

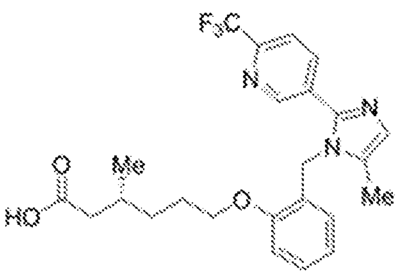

(hereinafter "Compound A")

reduced kidney injury in a rat model of acute kidney disease (see Example 2). Specifically, Compound A significantly reduced plasma creatinine levels, improves kidney function and reduces the early kidney injury biomarker NGAL, when administerd intravenously four hours after ischemic reperfusion injury was induced to the kidneys of laboratory rats.

It has also been found that Compound B, represented by the following structural formula:

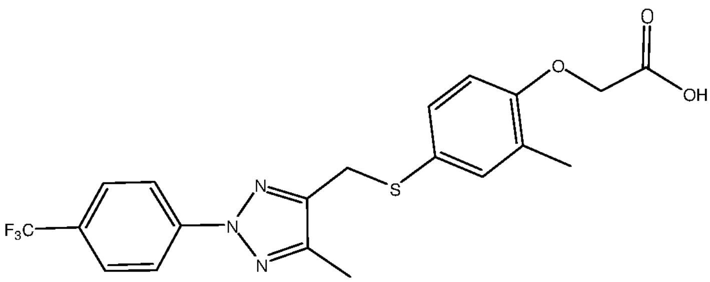

(hereinafter "Compound B")

reduced kidney injury in a rat model of acute kidney disease (see Example 3). Specifically, Compound B sigificantly reduced plasma creatinine levels and improves kidney function when administered orally four hours after ischemic

reperfusion injury was induced to the kidneys of laboratory rats.

Based on these discoveries, methods of treating acute kidney injury are disclosed herein.

Described is a method of treating a human patient with acute kidney injury. The method comprises intravenously administering to the patient an effective amount of Compound A or a pharmaceutically acceptable salt thereof.

Also described is a method of treating a human patient with acute kidney injury. The method comprises administering to the patient an effective amount of Compound B or a pharmaceutically acceptable salt thereof. In one embodiment, Compound B is administered orally; in another embodiment, it is administered intravenously.

Further described is a method of treating a human patient with acute kidney injury. The method comprises administering to the patient an effective amount of Compound C:

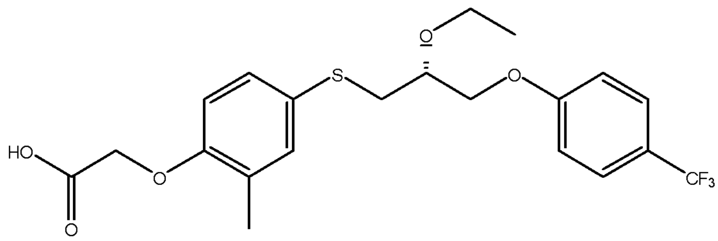

(hereinafter "Compound C");

or a pharmaceutically acceptable salt thereof. In one embodiment, Compound C is administered orally; in another embodiment, it is administered intravenously.

An embodiment of the invention is Compound A or a pharmaceutically acceptable salt thereof, for use in intravenously treating a human patient with acute kidney injury.

Also described is Compound B, or a pharmaceutically acceptable salt thereof, for use in treating a human patient with acute kidney injury. In one embodiment, Compound B is for oral administration; in another embodiment, Compound B is for intravenous administration.

Further described is Compound C, or a pharmaceutically acceptable salt thereof, for use in treating a human patient with acute kidney injury. In one embodiment, Compound C is for oral administration; in another embodiment, Compound C is for intravenous administration.

## BRIEF DESCRIPTION OF THE DRAWINGS

**Figure 1** a, b and c are bar graphs showing the therapeutic effect of Compound A administered intravenously in a rat model of acute kidney injury. The bar graph 1a shows the plasma creatinine levels in mg/dL in rats 24 hours after kidney injury. The bars from left to right represent plasma creatinine levels in rats with no kidney injury intravenously administered vehicle and in rats with kidney injury intravenously administered vehicle; 0.3 mpk of Compound A; 1 mpk of Compound A; and 3.0 mpk of Compound A. Similarly bar graph 1b shows the creatinine clearance levels, an estimation of kidney function or GFR (glomerular filtration rate) 24 hours after kidney injury and bar graph 1c shows the urinary levels of NGAL (Neutrophil Gelatinase-Associated Lipocalin) 48 hours after kidney injury. NGAL is an early kidney injury biomarker often used in the clinic.

**Figure 2** a and b are bar graphs showing the therapeutic effect of Compound B in a rat model of acute kidney injury. The bar graph 2a shows the plasma creatinine levels in mg/dL in rats 24 hours after kidney injury. The bars from left to right represent plasma creatinine levels in rats with sham surgery dosed with 30 mpk vehicle; rats with acute kidney injury dosed with 30 mpk vehicle; and rats with acute kidney injury dosed with 30 mpk of Compound B. Similarly bar graph 2b shows the creatinine clearance levels, an estimation of kidney function or GFR (glomerular filtration rate).

## DETAILED DESCRIPTION

Described herein are methods for treating acute kidney injury in a human patient. Such methods comprise administering an effective amount of Compound A, Compound B, or Compound C, or a pharmaceutically acceptable salt of Compound A, Compound B, or Compound C, to the human patient. In one embodiment, Compound A, Compound B, or Compound C, or a pharmaceutically acceptable salt of Compound A, Compound B, or Compound C, is administered after the human patient develops acute kidney injury. Compound A is preferably administered intravenously.

As used herein, the term "acute kidney injury" or "AKI", previously referred to as "acute renal failure" or "ARF", refers to an acute clinical syndrome characterized by rapid decline in renal functions, which is caused by a number of

factors such as a reduction in renal blood flow, glomerulonephritis, use of nephrotoxic antibiotics and anticancer agents.

Acute kidney injury is defined as an abrupt decrease in kidney function, and can be diagnosed based on a patient exhibit changes in one or more of serum creatinine level, glomerular filtration rate, or urine output. For example, acute kidney injury can be characterized by a serum creatinine level of at least 1.5 times baseline, wherein baseline refers to the patient's serum creatinine level no more than 7 days prior. For example, a patient having acute kidney injury can have a serum creatinine level is 1.5 to 1.9 times baseline, 2.0 to 2.9 times baseline, or 3.0 or more times baseline. Alternatively, acute kidney injury can be characterized by an increase in serum creatinine of at least 0.3 mg/dL or at least 0.4 mg/dL.

Alternatively, acute kidney injury can be characterized by a glomerular filtration rate of less than 90 mL/min/1.73 $m^2$. For example, a patient having acute kidney injury can have glomerular filtration rate of 60-89 mL/min/1.73 $m^2$, 30-59 mL/min/1.73 $m^2$, 15-29 mL/min/1.73 $m^2$, or less than 15 mL/min/1.73 $m^2$.

Alternatively, acute kidney injury can be characterized by the patient having a urine output of less than 0.5 mL/Kg over 6 hours, less than 0.5 mL/Kg over 12 hours, less than 0.3 mL/Kg over 12 hours, or anuria for 12 or more hours.

Acute kidney injury can occur with specific kidney diseases (*e.g.*, acute interstitial nephritis, acute glomerular and vasculitic renal diseases); non-specific conditions (*e.g.*, ischemia, toxic injury); as well as extrarenal pathology (*e.g.,* prerenal azotemia, and acute postrenal obstructive nephropathy). More than one of these conditions may coexist in the same patient and, more importantly, epidemiological evidence supports the notion that even mild, reversible AKI has important clinical consequences, including increased risk of death. Furthermore, because the manifestations and clinical consequences of AKI can be quite similar (even indistinguishable) regardless of whether the etiology is predominantly within the kidney or predominantly from outside stresses on the kidney, the syndrome of AKI encompasses both direct injury to the kidney as well as acute impairment of function.

In some embodiments, the patient being treated for AKI has diabetes, underlying renal insufficiency, nephritic syndrome, atherosclerotic disease, sepsis, hypotension, hypoxia, myoglobinuria-hematuria, or liver disease. In other embodiments, the patient is elderly, pregnant, a surgical patient, or has been exposed to a nephrotoxic agent.

In a specific embodiment, the patient being treated for AKI is a surgical patient. Accordingly, in certain embodiments, the compound is be administered to a surgical patient after surgery.

In another embodiment, the patient being treated for AKI has been exposed to a nephrotoxic agent. A nephrotoxic agent is a drug or chemical capable of causing AKI. Drugs or chemicals capable of causing acute kidney injury include, but are not limited to, cisplatin; gentamicin; cephaloridine; cyclosporine; amphotericin; carbon tetrachloride; trichloroethylene; and dichloroacetylene.

As such, a patient with AKI from any of the conditions mentioned in the previous three paragraphs can be treated with Compound A, Compound B, or Compound C, or a pharmaceutically acceptable salt of Compound A, Compound B, or Compound C, in accordance with the disclosed methods.

Pharmaceutically acceptable salts of Compound A, Compound B, and Compound C can be used in the disclosed methods. The term "pharmaceutically-acceptable salt" refers to pharmaceutical salts that are, within the scope of sound medical judgment, suitable for use in contact with the tissues of humans and lower animals without undue toxicity, irritation, and, allergic response, and are commensurate with a reasonable benefit/risk ratio. Pharmaceutically-acceptable salts are well known in the art. For example, S. M. Berge *et al.* describes pharmacologically acceptable salts in J. Pharm. Sci., 1977, 66, 1-19. Salts formed by addition with acids, *e.g.*, hydrochloric acid, hydrobromic acid, phosphoric acid, nitric acid, sulfuric acid, acetic acid, benzenesulfonic acid, benzoic acid, methanesulfonic acid, *p*-toluenesulfonic acid, and meglumine acid; and by addition with bases, *e.g.*, ammonium salts, alkali metal salts (such as sodium and potassium salts), alkaline earth metal salts (such as magnesium and calcium salts) and organic base salts (such as meglumine and L-lysine salts).

*Pharmaceutical Compositions and Administration Thereof*

Compound A, Compound B, or Compound C, or a pharmaceutically acceptable salt of Compound A, Compound B, or Compound C, can be used in combination with other agents known to have beneficial on AKI.

The precise amount of compound administered to provide an "effective amount" to the subject will depend on the mode of administration, the type, and severity of the disease and/ or condition and on the characteristics of the subject, such as general health, age, sex, body weight, and tolerance to drugs. The skilled artisan will be able to determine appropriate dosages depending on these and other factors. The term "effective amount" means an amount when administered to the subject which results in beneficial or desired results, including clinical results, *e.g.*, inhibits, suppresses or reduces the symptoms of the condition being treated in the subject as compared to a control. For example, an effective amount can be from 0.1 mg to about 50 g per day.

The terms "administer", "administering", "administration", and the like, as used herein, refer to methods that may be used to enable delivery of compositions to the desired site of biological action. These methods include, but are not limited to, intraarticular (in the joints), intravenous, intramuscular, intratumoral, intradermal, intraperitoneal, subcutaneous, orally, topically, intrathecally, inhalationally, transdermally, rectally, and the like. Oral and intravenous administration are commonly used, for example, when the condition being treated is acute kidney injury. Administration techniques that can be employed with the agents and methods described herein are found in *e.g.,* Goodman and Gilman, The Pharmacological Basis of Therapeutics, current ed.; Pergamon; and Remington's, Pharmaceutical Sciences (current edition), Mack Publishing Co., Easton, Pa.

Pharmaceutical compositions used in the disclosed methods include Compound A, Compound B, or Compound C, or a pharmaceutically acceptable salt of Compound A, Compound B, or Compound C, and typically at least one additional substance, such as an excipient, a known therapeutic other than those of the present disclosure, and combinations thereof.

The pharmaceutical compositions used in the disclosed methods are formulated to be compatible with its intended route of administration. In an embodiment, the composition is formulated in accordance with routine procedures as a pharmaceutical composition adapted for intravenous, subcutaneous, intramuscular, oral, intranasal, or topical administration to human beings.

Administration of therapeutic agents by intravenous formulation is well known in the pharmaceutical industry. Intravenous formulations comprise the pharmaceutically active agent (*e.g*., Compound A, Compound B, or Compound C, or a pharmaceutically acceptable salt of Compound A, Compound B, or Compound C) dissolved in a pharmaceutically acceptable solvent or solution, such as sterile water, normal saline solutions, lactated Ringer's, or other salt solutions such as Ringer's solution. The formulation should promote the overall stability of the active ingredient(s), also, the manufacture of the formulation should be cost-effective. All of these factors ultimately determine the overall success and usefulness of an intravenous formulation.

An oral formulation typically is prepared as a compressed preparation in, for example, the form of a tablet or pill. A tablet may contain, for example about 5-10% of the active ingredient (*e.g.*, Compound A, Compound B, or Compound C, or a pharmaceutically acceptable salt of Compound A, Compound B, or Compound C) about 80% of fillers, disintegrants, lubricants, glidants, and binders; and 10% of compounds which ensure easy disintegration, disaggregation, and dissolution of the tablet in the stomach or the intestine. Pills can be coated with sugar, varnish, or wax to disguise the taste.

## EXEMPLIFICATION

### Example 1

**Synthesis** of **(*R*)-3-methyl-6-(2-((5-methyl-2-(6-(trifluoromethyl)pyridin-3-yl)-1*H*-imidazol-l-yl)methyl)phenoxy)hexanoic acid (Compound A)**

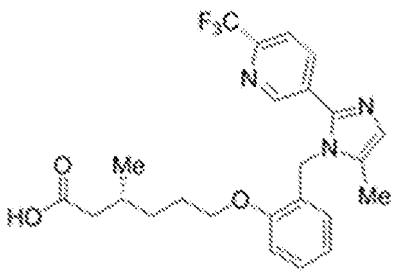

**Scheme:**

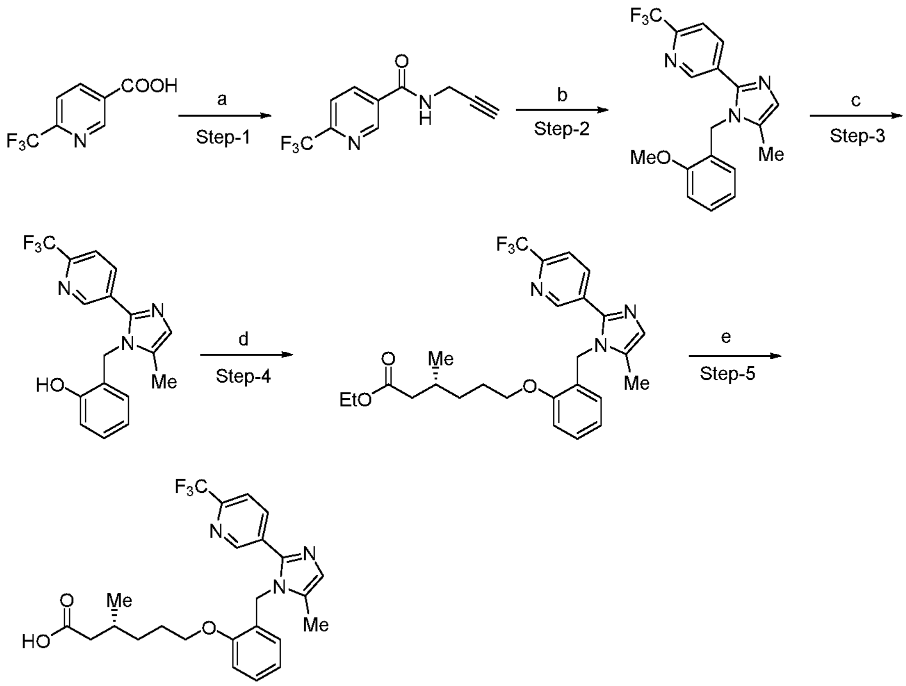


*Synthesis of ethyl (R)-6-bromo-3-methylhexanoate:*

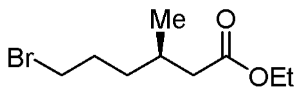

In a 1 L round bottom flask, a solution of ethyl (*R*)-6-hydroxy-3-methylhexanoate (65.0 g, 373.56 mmol) in dichrolomethane (650 mL) was treated with $PBr_3$ (101.0 g, 373.56 mmol) at room temperature (RT). The reaction mixture was stirred at RT for 3 h. Upon completion of reaction (monitored by TLC), the reaction mixture was diluted with water (500 mL) and extracted with diethyl ether (3 x 500 mL). The organic extract was separated and dried over anhydrous $Na_2SO_4$. The solvent was removed under reduced pressure to get the title compound (57.12 g).

*Step-1: Synthesis of N-(prop-2-yn-1-yl)-6-(trifluoromethyl)nicotinamide:*

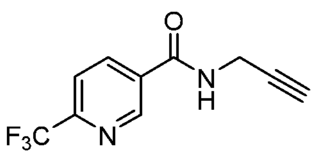

In a 100 mL round bottom flask, a stirred solution of 6-(trifluoromethyl)nicotinic acid (3 g, 15.70 mmol) and prop-2-yn-1-amine (1.05 g, 18.84 mmol) in dimethylformamide (DMF: 50 mL) was treated with 1-[bis(dimethylamino)methylene]-1H-1,2,3-triazolo[4,5-b]pyridinium 3-oxide hexafluorophosphate(7.2 g, 18.84 mmol) and triethylamine (3.1 mL, 23.55 mmol) at RT under nitrogen atmosphere. The resulting reaction mixture was stirred at RT for 3 h. Upon completion of reaction (monitored by TLC), the reaction mixture was diluted with cold water and solid precipitated was filtered, washed with water and dried under reduced pressure to get the title compound (2.6 g, 72.6 %).
$^1H$ NMR (300 MHz, $CDCl_3$): δ 9.08 (d, *J* = 2.1 Hz, 1H), 8.32 (dd, *J* = 8.4, 2.4 Hz, 1H), 7.78 (d, *J* = 7.8 Hz, 1H), 6.62 (brs, 1H), 4.30-4.28 (m, 2H), 2.33 (t, *J* = 2.4 Hz, 1H).
LCMS (ESI+, *m/z*): 229.2 $(M+H)^+$.

*Step-2: Synthesis of 5-(1-(2-methoxybenzyl)-5-methyl-1H-imidazol-2-yl)-2-(trifluoromethyl) pyridine:*

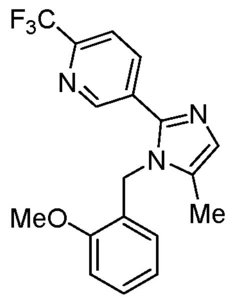

In a 50 mL resealable tube, a solution of N-(prop-2-yn-1-yl)-6-(trifluoromethyl)nicotinamide (1.0 g, 4.38 mmol) and 2-methoxyphenybenzyl amine (1.2 g, 8.77 mmol) in toluene (10 mL) was treated with zinc trifluoromethanesulfonate (0.16 g, 0.43 mmol) at RT under nitrogen atmosphere. The reaction mixture was heated at 120 °C for 12 h. Upon completion of reaction (monitored by TLC), the reaction mixture was diluted with water and extracted with EtOAc (3 x 20 mL). The organic extract was washed with saturated $NaHCO_3$, brine and dried over anhydrous $Na_2SO_4$. The solution was concentrated under reduced pressure and residue obtained was purified by silica gel column chromatography (elution, 25% EtOAc in hexanes) to yield the title compound (0.8 g, 52.6%).
$^1$H NMR (400 MHz, $CDCl_3$): δ 8.79 (s, 1H), 8.07 (d, *J* = 8.1 Hz, 1H), 7.68 (d, *J* = 8.1 Hz, 1H), 7.31 (t, *J* = 8.4 Hz, 1H), 7.09 (s, 1H), 6.94-6.87 (m, 2H), 6.56 (d, *J* = 7.5 Hz, 1H), 5.16 (s, 2H), 3.87 (s, 3H). LCMS (ESI+, *m/z*): 348.3 $(M+H)^+$.

*Step-3: Synthesis of 2-((5-methyl-2-(6-(trifluoromethyl)pyridin-3-yl)-1H-imidazol-1-yl) methyl)phenol:*

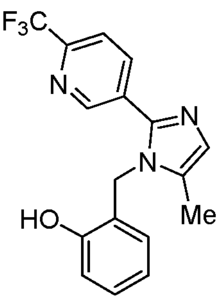

In a 100 mL round bottom flask, a solution of 5-(1-(2-methoxybenzyl)-5-methyl-1*H*-imidazol-2-yl)-2-(trifluoromethyl)pyridine (0.8 g, 2.31 mmol) in dichloromethane (300 mL) was treated with $BBr_3$ (0.8 mL, 2.31 mmol) drop wise at 0 °C. The reaction mixture was stirred at RT for 2 h. Upon completion of reaction (monitored by TLC), the reaction mixture was basified (pH ~ 9) with aqueous $NaHCO_3$ and extracted with ethyl acetate (EtOAc). The organic extract was dried over anhydrous $Na_2SO_4$ and concentrated under reduced pressure to afford the title compound (0.5 g, 65.1%)
$^1$H NMR (400 MHz, DMSO-$d_6$): δ 9.92 (s, 1H), 8.83 (s, 1H), 8.12 (d, *J* = 8.1 Hz, 1H), 7.94 (d, *J* = 8.1 Hz, 1H), 7.12 (d, *J* = 6.9 Hz, 1H), 7.02 (s, 1H), 6.87 (d, *J* = 7.8 Hz 1H), 6.73 (t, *J* = 7.2 Hz, 1H), 6.37 (d, *J* = 7.2 Hz, 1H), 5.20 (s, 2H), 2.15 (s, 3H). LCMS (ESI+, m/z): 334.3 $(M+H)^+$.

*Step-4: Synthesis of ethyl (R)-3-methyl-6-(2-((5-methyl-2-(6-(trifluoromethyl)pyridin-3-yl)-1H-imidazol-1-yl)methyl)phenoxy)hexanoate:*

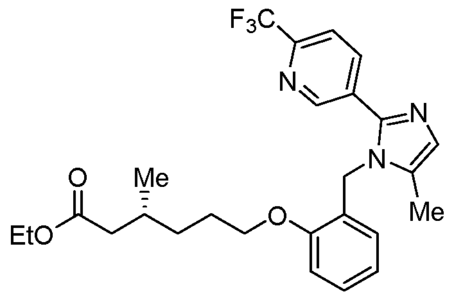

In a 50 mL round bottom flask, a stirred solution of 2-((5-methyl-2-(6-(trifluoromethyl)pyridin-3-yl)-1*H*-imidazol-1-yl)methyl)phenol (0.5 g, 1.50 mmol) in DMF (10 mL) was treated with $K_2CO_3$ (0.41 g, 3.00 mmol) and ethyl (*R*)-6-bromo-3-methylhexanoate (0.710 g, 3.00 mmol) at RT under nitrogen atmosphere. The resulting reaction mixture was heated 60 °C for 12 h. Upon completion of the reaction (monitored by TLC), the reaction mixture was quenched with ice cold water and extracted with ethyl acetate (75 mL X 3). The combined organic extract was washed with brine, dried over anhydrous $Na_2SO_4$ and concentrated under reduced pressure. The residue obtained was purified by silica gel column chromatography (gradient elution, 15-30% EtOAc in hexanes) to afford the title compound (0.45 g, 61.3%).
Yield: 0.45 g (61.3%).
LCMS (ESI+, *m/z*): 491.0 $(M+H)^+$.

*Step-5: Synthesis of (R)-3-methyl-6-(2-((5-methyl-2-(6-(trifluoromethyl)pyridin-3-yl)-1H-imidazol-1-yl)methyl)phenoxy)hexanoic acid (Compound A):*

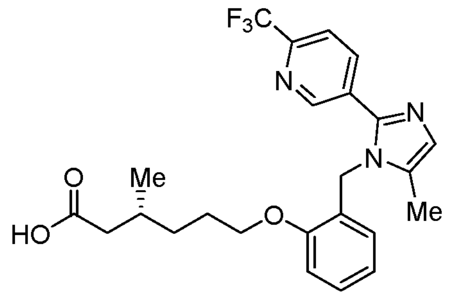

In a 250 mL round bottom flask, a stirred solution of ethyl (*R*)-3-methyl-6-(2-((5-methyl-2-(6-(trifluoromethyl)pyridin-3-yl)-1*H*-imidazol-1-yl)methyl)phenoxy)hexanoate (0.45 g, 0.92 mmol) in tetrahydrofuran (THF: 5 mL), ethanol (2.5 mL) and water (2.5 mL) was treated with lithium hydroxide monohydrate (16.0 g, 74.33 mmol) at RT. The reaction mixture was stirred at RT for 12 h. Upon completion of reaction (monitored by TLC), the reaction mixture was concentrated under reduced pressure. The residue obtained was washed with EtOAc, diluted with cold water and acidified (pH ~5) with 1N HCl. The solid was filtered and dried under reduced pressure to give the title compound (0.166 g, 39.2 %).
$^1$H NMR (400 MHz, DMSO-$d_6$): δ11.96 (brs, 1H), 8.79 (s, 1H), 8.05 (d, *J* = 8.0 Hz, 1H), 7.90 (d, *J* = 8.0 Hz, 1H), 7.24 (t, *J* = 7.6 Hz, 1H), 7.02 (d, *J* = 8.4 Hz, 1H), 7.00 (s, 1H), 6.84 (t, *J* = 7.6 Hz, 1H), 6.43 (d, *J* = 7.2 Hz, 1H), 5.21 (s, 2H), 3.98 (t, *J* = 6.0 Hz, 2H), 2.19-2.14 (m, 1H), 2.13 (s, 3H), 2.03-1.94 (m, 1H), 1.85-1.80 (m, 1H), 1.68-1.66 (m, 2H), 1.38-1.36 (m, 1H), 1.28-1.18 (m, 1H), 0.85 (d, *J* = 6.4 Hz, 3H).
$^{19}$F NMR (400 MHz, DMSO-$d_6$): δ -66.46
LCMS (ESI+, *m/z*): 462.3 $(M+H)^+$.
HPLC: 95.11 % (210 nm).

**Preparation of Meglumine Salt of Compound A**

Two separate methods were used to generate a meglumine salt of Compound A.

*Method 1*

Compound A (102.7 mg) was combined with meglumine (43.7 mg) and 2 mL of 2-propanol in a 4 mL glass

vial. The vial was sealed with a cap and the contents were subjected to sonication at 25 °C for 20 minutes followed by stirring at 50 °C for 60 minutes. The vial was then moved to a new stir plate and the slurry in the vial was stirred at 25 °C.

*Method 2*

Compound A (102.2 mg) was combined with meglumine (43.2 mg) and 2 mL of acetonitrile in a 4 mL glass vial. The vial was sealed with a cap and the contents were subjected to sonication at 25 °C for 20 minutes followed by stirring at 50 °C for 60 minutes. The vial was then moved to a new stir plate and the slurry in the vial was stirred at 25 °C.

For both method 1 and method 2, after 2 days of stirring at 25 °C, both samples were centrifuged, supernatants discarded, and solids were air dried.

**Preparation of Hydrate of Meglumine Salt of Compound A**

In a 500 mL round bottom flask, a stirred solution of Compound A (20 g, 43.33 mmol) in THF (100 mL) and water (100 mL) was treated meglumine (8.45 g, 43.33 mmol) at 0 °C. The resulting reaction mixture was stirred at RT for 6 h. The reaction mixture was concentrated under reduced pressure and solid obtained was dried under reduced pressure (3h) to afford the title compound as a white solid (28.5 g, 98.95%).

$^1H$ NMR (400 MHz, $CD_3OD$): $\delta$ 8.75 (s, 1H), 8.02 (d, $J$ = 8.4 Hz, 1H), 7.82 (d, $J$ = 8.0 Hz 1H), 7.26 (t, $J$ = 8.4 Hz, 1H), 7.03 (s, 1H), 6.99 (d, $J$ = 8 Hz, 1H), 6.85 (t, $J$ = 7.6 Hz, 1H), 6.50 (d, $J$ = 7.6 Hz, 1H), 5.25 (s, 2H), 4.09-3.99 (m, 3H), 3.97-3.77 (m, 2H), 3.74-3.61 (m, 3H), 3.29-3.06 (m, 2H), 2.64 (s, 3H), 2.22 (s, 3H), 2.18-2.14 (m, 1H), 1.99 - 1.94 (m, 2H), 1.83 - 1.75 (m, 2H), 1.51 - 1.38 (m, 1H), 1.32-1.22 (m, 1H), 0.86 (d, $J$ = 6.0 Hz, 3H).

$^{19}F$ NMR (400 MHz, $CD_3OD$): $\delta$ -69.39

Elemental Analysis: Calcd for $C_{31}H_{43}F_3N_4O_8$. $H_2O$: C, 55.18; H, 6.72; N, 8.30. Found: C, 54.95; H, 6.89; N, 8.07

Moisture Content (Karl Fischer): 2.33%

**Example 2**

**Compound A Reduces Ischemia Reperfusion Injury When Administered Intravenously in an Animal Model of Acute Kidney Injury**

*Animals, surgery and dosing:* Sprague-Dawley male rats approximately 280-300g, with ad libitum access to standard feed and water were used in these experiments. Rats (n=8/group) were anesthetized with isoflurane and placed ventrally on a temperature controlled heated surgical platform. A skin incision was made on the dorsal surface, exposing both kidneys through flank incisions. Vascular clips were applied to both renal pedicles and occlusion lasted 45 minutes. After 45 min, the clips were removed, kidneys were monitored for successful reperfusion, and surgical sites were then sutured. The sham group (n=4 rats) was subjected to similar surgical procedures, except that the occluding clamps were not applied. Compound A was formulated as a fresh daily clear solution of the hydrate of the meglumine salt of Compound A (1.5 Molar equivalent of Compound A) in 5% dextrose. Compound A was dosed IV via tail vein at 3 mg/kg, 1 mg/kg or 0.3 mg/kg 4 hours after animals awoke from surgery and sham surgery and IRI control animals were similarly dosed with vehicle. *Blood collection:* Twenty-four (24) hours after reperfusion, blood was collected in K2 EDTA tubes by retro-orbital bleeding from all groups under mild isoflurane anesthesia. Plasma was separated by centrifugation at 3000 rpm for 10 minutes at 4 °C.

*Urine collection:* all study animal groups were placed in metabolic cages immediately after surgery recovery and urine was collected for 24 or 48 hours. Urine volume was carefully measured and then was centrifuged at 3000 rpm for 10 min at 4 °C to remove sediments. Plasma and urine creatinine were analyzed using a fully automated clinical biochemistry analyzer (Siemens Dimension ® Xpand ® Plus Integrated Chemistry System). Urinary NGAL was analyzed using a BioPorto ELISA kit. Creatinine clearance was calculated as follows:

$$Creatinine\ Clearance = \left(\frac{\left(Urinary\ Creatinine\ \left(\frac{mg}{dL}\right) X\ Urine\ Volume(mL)\right)}{\left(Serum\ Creatinine\ \left(\frac{mg}{dL}\right) X\ 1440\ mins\ (24\ hr)\right)}\right)/(100g\ BodyWeight)$$

*Data Analysis and Statistical analysis:*

GraphPad Prism software, Version 6.05 was used for graphing and statistical testing. Creatinine was tested

for normal distribution in all groups via a D'Agostino-Pearson omnibus normality test and a Shapiro-Wilk normality test. Statistical significance ($p<0.05$) is determined by an ordinary One-way ANOVA followed by Dunnett's multiple comparison using the IRI-treated group as the control group. ** $p<0.01$, *** $p<0.001$ and **** $p<0.0001$ vs. IR-Vehicle.

*Results:* The data is shown in Figure 1. Compound A, dosed IV 4 hours after ischemia, reduces kidney injury. Compound A significantly reduces plasma creatinine, improves kidney function and reduces the early kidney injury biomarker NGAL when dosed at 0.3 mg/kg, 1 mg/kg or

3 mg/kg.

**Example 3**

**Compound B Reduces Ischemia Reperfusion Injury When Administered Orally in an Animal Model of Acute Kidney Injury**

*Animals, surgery and dosing:* Sprague-Dawley male rats approximately 280-300g, with ad libitum access to standard feed and water were used in these experiments. Rats (n=8/group) were anesthetized with isoflurane and placed ventrally on a temperature controlled heated surgical platform. A skin incision was made on the dorsal surface, exposing both kidneys through flank incisions. Vascular clips were applied to both renal pedicles and occlusion lasted 45 minutes. After 45 min, the clips were removed, kidneys were monitored for successful reperfusion, and surgical sites were sutured. The sham group (n=4 rats) was subjected to similar surgical procedures, except that the occluding clamps were not applied. Compound B was formulated as a fresh daily suspension in 0.25% sodium carboxymethylcellulose, 0.25% Tween-80 in purified water. Compound B was dosed orally at 30 mg/kg 4 hours after animals awoke from surgery and sham surgery and IRI control animals were similarly dosed with vehicle.

*Blood collection:* Twenty-four (24) hours after reperfusion, blood was collected in K2 EDTA tubes by retro-orbital bleeding from all groups under mild isoflurane anesthesia. Plasma was separated by centrifugation at 3000 rpm for 10 minutes at 4 °C.

*Urine collection:* all study animal groups were placed in metabolic cages immediately after surgery recovery and urine was collected for 24 hours. Urine volume was carefully measured and then was centrifuged at 3000 rpm for 10 min at 4 °C to remove sediments.

Plasma and urine creatinine were analyzed using a fully automated clinical biochemistry analyzer (Siemens Dimension ® Xpand ® Plus Integrated Chemistry System). Creatinine clearance was calculated as follows:

$$Creatinine\ Clearance = \left(\frac{\left(Urinary\ Creatinine\ \left(\frac{mg}{dL}\right) X\ Urine\ Volume(mL)\right)}{\left(Serum\ Creatinine\ \left(\frac{mg}{dL}\right) X\ 1440\ mins\ (24\ hr)\right)}\right)/(100g\ BodyWeight)$$

*Data Analysis and Statistical analysis:*

GraphPad Prism software, Version 6.05 was used for graphing and statistical testing. Data was tested for normal distribution in all groups via a D'Agostino-Pearson omnibus normality test and a Shapiro-Wilk normality test. Normally distributed data was subjected to an unpaired, two-tailed t test. Non-normally distributed data was subjected to a Mann-Whitney test (non-parametric). Statistical significance is determined by $p<0.05$ of IRI-vehicle compared to compound treated groups. ** $p<0.01$ and *** $p<0.001$ vs. IR-Vehicle.

*Results:* Compound B, dosed 4 hours after ischemia, reduces kidney injury. Compound B (Figure 2) reduces plasma creatinine and improves kidney function when administered orally. The bar graph a shows the plasma creatinine levels in mg/dL in rats 24 hours after kidney injury reduce plasma creatinine when administered orally. The bars from left to right represent plasma creatinine levels in rats with sham surgery dosed with vehicle; rats with acute kidney injury dosed with vehicle; and rats with acute kidney injury dosed with 30 mpk of Compound B (Figure 2 a). Similarly bar graph b shows the creatinine clearance levels, an estimation of kidney function or GFR (glomerular filtration rate) (Figure 2 b)

**Claims**

**1.** A compound represented by the following structural formula:

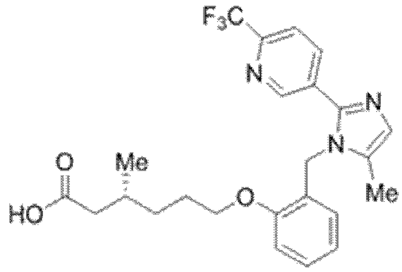

;

or a pharmaceutically acceptable salt thereof, for use in intravenously treating a human patient with acute kidney injury.

2. The compound for use according to claim 1, wherein the pharmaceutical acceptable salt of the compound is a meglumine salt.

3. The compound or pharmaceutically acceptable salt thereof for use of claim 1 or claim 2, wherein the patient is a surgical patient and the compound or the pharmaceutically acceptable salt thereof is administered to the patient after surgery.

4. The compound or the pharmaceutically acceptable salt thereof for use of claims 1 to 3, wherein the compound or the pharmaceutically acceptable salt thereof is administered in an effective amount from 0.1 mg to about 50 g per day.

5. The compound or the pharmaceutically acceptable salt thereof for use of any one of claims 1 to 4, wherein the patient is being treated for acute kidney injury from acute interstitial nephritis.

6. The compound or the pharmaceutically acceptable salt thereof for use of any one of claims 1 to 4, wherein the patient is being treated for acute kidney injury from glomerular renal disease.

7. The compound or the pharmaceutically acceptable salt thereof for use of any one of claims 1 to 4, wherein the patient is being treated for acute kidney injury from acute vasculitic renal disease.

8. The compound or the pharmaceutically acceptable salt thereof for use of any one of claims 1 to 4, wherein the patient is being treated for acute kidney injury from ischemia.

9. The compound or the pharmaceutically acceptable salt thereof for use of any one of claims 1 to 4, wherein the patient is being treated for acute kidney injury from toxic injury.

10. The compound or the pharmaceutically acceptable salt thereof for use of any one of claims 1 to 4, wherein the patient is being treated for acute kidney injury from prerenal azotemia.

11. The compound or the pharmaceutically acceptable salt thereof for use of any one of claims 1 to 4, wherein the patient is being treated for acute kidney injury from acute postrenal destructive nephropathy.

12. The compound or the pharmaceutically acceptable salt thereof for use of any one of claims 1 to 4, wherein the patient is being treated for acute kidney injury from diabetes, underlying renal insufficiency, nephritic syndrome, atherosclerotic disease, sepsis, hypotension, hypoxia, myoglobinuria-hematuria, or liver disease.

13. The compound or the pharmaceutically acceptable salt thereof for use of any one of claims 1 to 4, wherein the patient being treated for acute kidney injury is elderly, pregnant, a surgical patient, or has been exposed to a nephrotoxic agent.

14. The compound or the pharmaceutically acceptable salt thereof for use of any one of claims 1 to 4, wherein the patient has been exposed to a nephrotoxic agent and the nephrotoxic agent is a drug or chemical capable of causing acute kidney injury.

15. The compound or the pharmaceutically acceptable salt thereof for use of claim 14, wherein the drug or chemical is one or more of the compounds selected from the group consisting of cisplatin; gentamicin; cephaloridine; cyclosporine; amphotericin; carbon tetrachloride; trichloroethylene; and dichloroacetylene.

**Patentansprüche**

1. Verbindung, dargestellt durch die folgende Strukturformel:

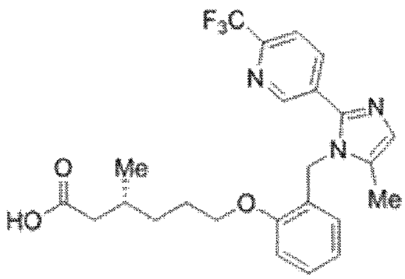

;

oder ein pharmazeutisch annehmbares Salz davon zur Verwendung bei der intravenösen Behandlung eines menschlichen Patienten mit akuter Nierenverletzung.

2. Verbindung zur Verwendung nach Anspruch 1, wobei das pharmazeutisch annehmbare Salz der Verbindung ein Megluminsalz ist.

3. Verbindung oder pharmazeutisch annehmbares Salz davon zur Verwendung nach Anspruch 1 oder Anspruch 2, wobei der Patient ein chirurgischer Patient ist und wobei die Verbindung oder das pharmazeutisch annehmbare Salz davon dem Patienten nach einer Operation verabreicht wird.

4. Verbindung oder pharmazeutisch annehmbares Salz davon zur Verwendung nach den Ansprüchen 1 bis 3, wobei die Verbindung oder das pharmazeutisch annehmbare Salz davon in einer wirksamen Menge von 0,1 mg bis etwa 50 g pro Tag verabreicht wird.

5. Verbindung oder pharmazeutisch annehmbares Salz davon zur Verwendung nach einem der Ansprüche 1 bis 4, wobei der Patient wegen einer akuten Nierenverletzung durch akute interstitielle Nephritis behandelt wird.

6. Verbindung oder pharmazeutisch annehmbares Salz davon zur Verwendung nach einem der Ansprüche 1 bis 4, wobei der Patient wegen einer akuten Nierenverletzung aufgrund einer glomerulären Nierenerkrankung behandelt wird.

7. Verbindung oder pharmazeutisch annehmbares Salz davon zur Verwendung nach einem der Ansprüche 1 bis 4, wobei der Patient wegen einer akuten Nierenverletzung aufgrund einer akuten vaskulitischen Nierenerkrankung behandelt wird.

8. Verbindung oder pharmazeutisch annehmbares Salz davon zur Verwendung nach einem der Ansprüche 1 bis 4, wobei der Patient wegen einer akuten Nierenverletzung durch Ischämie behandelt wird.

9. Verbindung oder pharmazeutisch annehmbares Salz davon zur Verwendung nach einem der Ansprüche 1 bis 4, wobei der Patient wegen einer akuten Nierenschädigung aufgrund einer toxischen Verletzung behandelt wird.

10. Verbindung oder pharmazeutisch annehmbares Salz davon zur Verwendung nach einem der Ansprüche 1 bis 4, wobei der Patient wegen einer akuten Nierenverletzung durch prärenale Azotämie behandelt wird.

11. Verbindung oder pharmazeutisch annehmbares Salz davon zur Verwendung nach einem der Ansprüche 1 bis 4, wobei der Patient wegen einer akuten Nierenverletzung aufgrund einer akuten postrenalen destruktiven Nephropathie behandelt wird.

12. Verbindung oder pharmazeutisch annehmbares Salz davon zur Verwendung in einem der Ansprüche 1 bis 4, wobei der Patient wegen einer akuten Nierenverletzung aufgrund von Diabetes, zugrunde liegender Niereninsuffizienz,

nephritischem Syndrom, atherosklerotischer Erkrankung, Sepsis, Hypotonie, Hypoxie, Myoglobinurie-Hämaturie oder Lebererkrankung behandelt wird.

**13.** Verbindung oder pharmazeutisch annehmbares Salz davon zur Verwendung nach einem der Ansprüche 1 bis 4, wobei der Patient, der wegen einer akuten Nierenverletzung behandelt wird, ein älterer Mensch, schwanger, ein chirurgischer Patient oder ein Patient ist, der mit einem nephrotoxischen Mittel in Kontakt gekommen ist.

**14.** Verbindung oder pharmazeutisch annehmbares Salz davon zur Verwendung nach einem der Ansprüche 1 bis 4, wobei der Patient mit einem nephrotoxischen Mittel in Kontakt gekommen ist und wobei das nephrotoxische Mittel ein Medikament oder eine Chemikalie ist, das/die eine akute Nierenschädigung verursachen kann.

**15.** Verbindung oder pharmazeutisch annehmbares Salz davon zur Verwendung nach Anspruch 14, wobei das Medikament oder die Chemikalie eine oder mehrere der Verbindungen ist, die ausgewählt sind aus der Gruppe, die aus Cisplatin, Gentamicin, Cephaloridin, Cyclosporin, Amphotericin, Tetrachlorkohlenstoff, Trichlorethylen und Dichloracetylen besteht.

## Revendications

**1.** Composé représenté par la formule développée suivante :

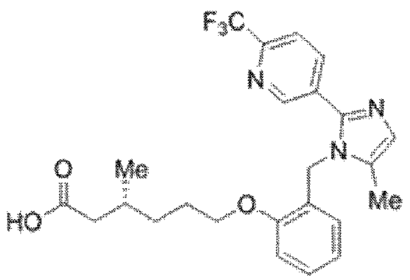

;

ou sel pharmaceutiquement acceptable de celui-ci, destiné à être utilisé pour le traitement intraveineux d'un patient humain souffrant d'une lésion rénale aiguë.

**2.** Composé destiné à être utilisé selon la revendication 1, dans lequel le sel pharmaceutiquement acceptable du composé est un sel de méglumine.

**3.** Composé ou sel pharmaceutiquement acceptable de celui-ci destiné à être utilisé selon la revendication 1 ou la revendication 2, dans lequel le patient est un patient chirurgical et le composé ou le sel pharmaceutiquement acceptable de celui-ci est administré au patient après la chirurgie.

**4.** Composé ou sel pharmaceutiquement acceptable de celui-ci destiné à être utilisé selon les revendications 1 à 3, dans lequel le composé ou le sel pharmaceutiquement acceptable de celui-ci est administré en une quantité efficace de 0,1 mg à environ 50 g par jour.

**5.** Composé ou sel pharmaceutiquement acceptable de celui-ci destiné à être utilisé selon l'une quelconque des revendications 1 à 4, dans lequel le patient est traité pour une lésion rénale aiguë due à une néphrite interstitielle aiguë.

**6.** Composé ou sel pharmaceutiquement acceptable de celui-ci destiné à être utilisé selon l'une quelconque des revendications 1 à 4, dans lequel le patient est traité pour une lésion rénale aiguë due à une maladie rénale glomérulaire.

**7.** Composé ou sel pharmaceutiquement acceptable de celui-ci destiné à être utilisé selon l'une quelconque des revendications 1 à 4, dans lequel le patient est traité pour une lésion rénale aiguë due à une maladie rénale vasculitique aiguë.

**8.** Composé ou sel pharmaceutiquement acceptable de celui-ci destiné à être utilisé selon l'une quelconque des

revendications 1 à 4, dans lequel le patient est traité pour une lésion rénale aiguë due à une ischémie.

9. Composé ou sel pharmaceutiquement acceptable de celui-ci destiné à être utilisé selon l'une quelconque des revendications 1 à 4, dans lequel le patient est traité pour une lésion rénale aiguë due à une lésion toxique.

10. Composé ou sel pharmaceutiquement acceptable de celui-ci destiné à être utilisé selon l'une quelconque des revendications 1 à 4, dans lequel le patient est traité pour une lésion rénale aiguë due à une azotémie prérénale.

11. Composé ou sel pharmaceutiquement acceptable de celui-ci destiné à être utilisé selon l'une quelconque des revendications 1 à 4, dans lequel le patient est traité pour une lésion rénale aiguë due à une néphropathie destructrice post-rénale aiguë.

12. Composé ou sel pharmaceutiquement acceptable de celui-ci destiné à être utilisé selon l'une quelconque des revendications 1 à 4, dans lequel le patient est traité pour une lésion rénale aiguë due au diabète, à une insuffisance rénale sous-jacente, au syndrome néphrétique, à une maladie athérosclérotique, à une septicémie, à une hypotension, à une hypoxie, à une myoglobinurie-hématurie ou à une maladie du foie.

13. Composé ou sel pharmaceutiquement acceptable de celui-ci destiné à être utilisé selon l'une quelconque des revendications 1 à 4, dans lequel le patient traité pour une lésion rénale aiguë est une personne âgée, enceinte, une patiente chirurgicale ou ayant été exposée à un agent néphrotoxique.

14. Composé ou sel pharmaceutiquement acceptable de celui-ci destiné à être utilisé selon l'une quelconque des revendications 1 à 4, dans lequel le patient a été exposé à un agent néphrotoxique et l'agent néphrotoxique est un médicament ou un produit chimique pouvant provoquer une lésion rénale aiguë.

15. Composé ou sel pharmaceutiquement acceptable de celui-ci destiné à être utilisé selon la revendication 14, dans lequel le médicament ou produit chimique est un ou plusieurs des composés choisis dans le groupe constitué par le cisplatine ;

la gentamicine ;
la céphaloridine ;
la cyclosporine ;
l'amphotéricine ;
le tétrachlorure de carbone ;
le trichloréthylène ; et
le dichloroacétylène.

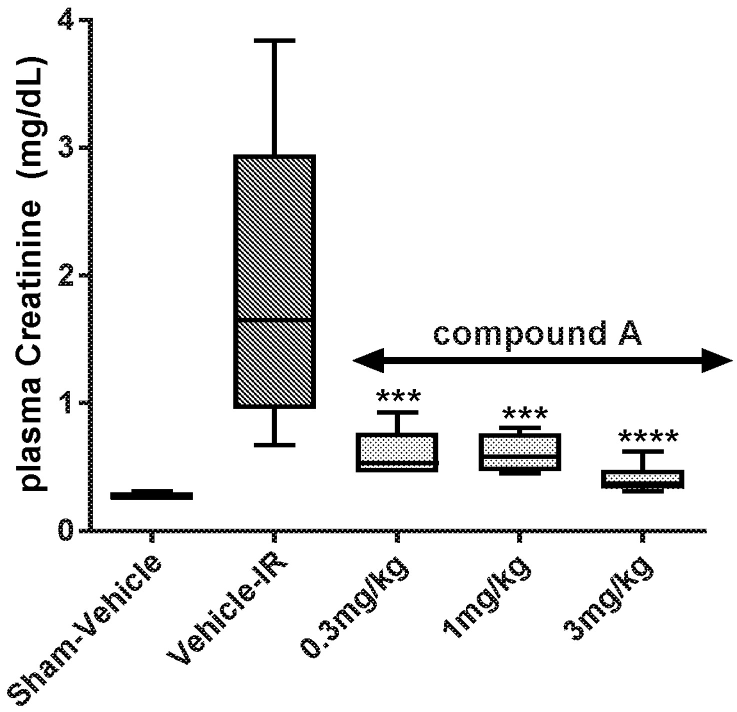
plasma Creatinine (mg/dL)
4
3
2
1
0
compound A
***
***
****
Sham-Vehicle
Vehicle-IR
0.3mg/kg
1mg/kg
3mg/kg

Figure 1a

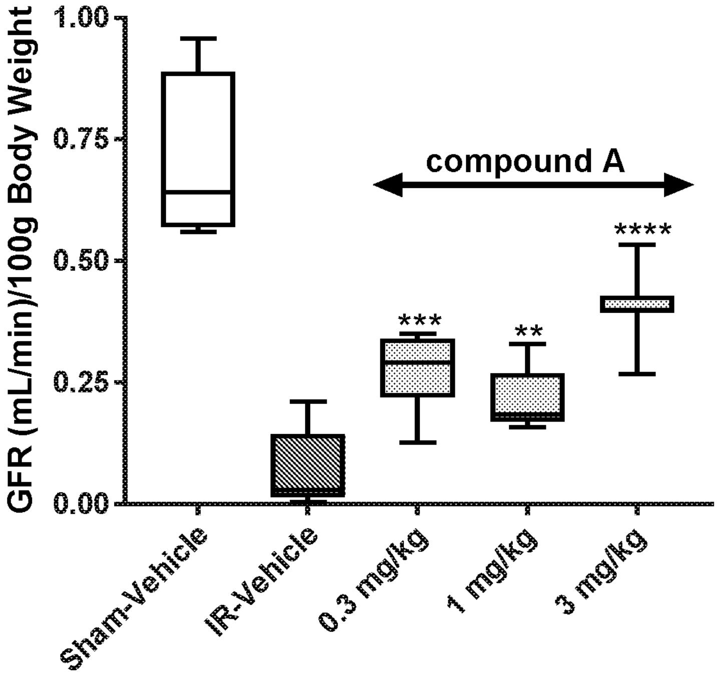
GFR (mL/min)/100g Body Weight
1.00
0.75
0.50
0.25
0.00
compound A
***
**
****
Sham-Vehicle
IR-Vehicle
0.3 mg/kg
1 mg/kg
3 mg/kg

Figure 1b

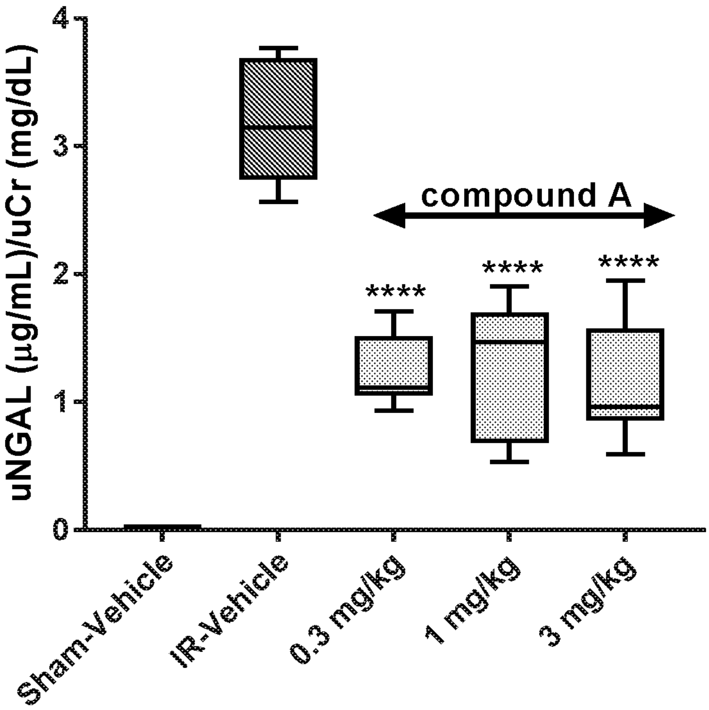
uNGAL (µg/mL)/uCr (mg/dL)
4
3
2
1
0
compound A
****
****
****
Sham-Vehicle
IR-Vehicle
0.3 mg/kg
1 mg/kg
3 mg/kg

Figure 1c

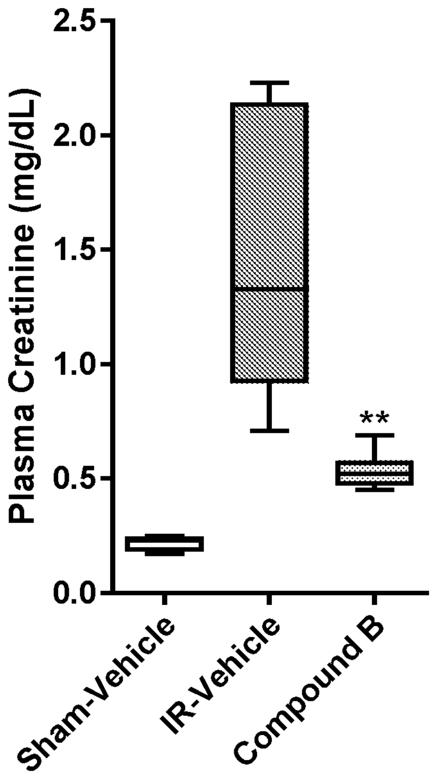
Plasma Creatinine (mg/dL)
2.5
2.0
1.5
1.0
0.5
0.0
**
Sham-Vehicle
IR-Vehicle
Compound B

Figure 2a

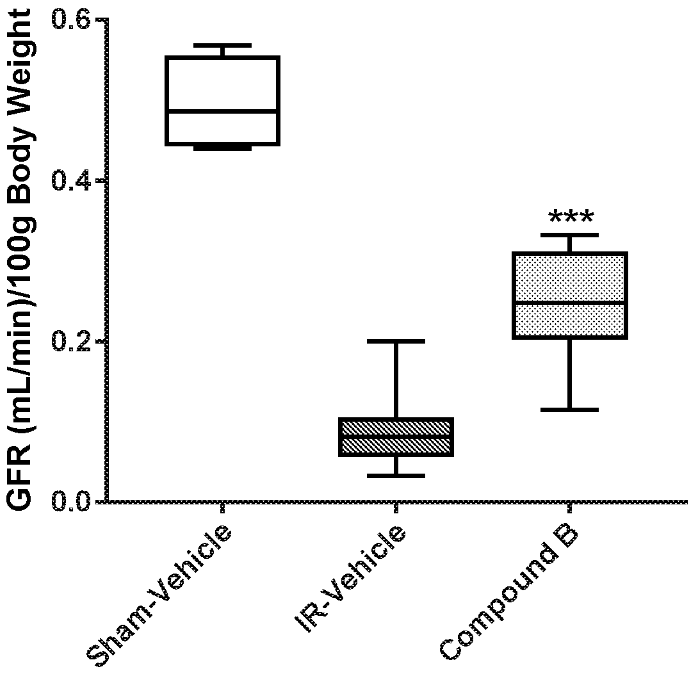
GFR (mL/min)/100g Body Weight
0.6
0.4
0.2
0.0
***
Sham-Vehicle
IR-Vehicle
Compound B

Figure 2b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **FEDOROVA et al.** *PLOS ONE,* 2013, vol. 8, e64436
- **EUN YOUNG LEE et al.** *Nephrol. Dial. Transplant.,* 2012, vol. 27, 4069-4079
- *J. Pharm. Sci.,* 1977, vol. 66, 1-19
- **GOODMAN ; GILMAN.** The Pharmacological Basis of Therapeutics. Pergamon
- Remington's, Pharmaceutical Sciences. Mack Publishing Co,